# EUROPEAN PATENT APPLICATION

(11) **EP 4 467 068 A1**
(43) Date of publication of application: **27.11.2024**
(21) Application number: 23175622.2
(22) Date of filing: 26.05.2023
(51) Int. Cl.: A61B 5/0205, A61B 5/00, A61B 5/087, A61B 5/11, A61B 5/113, A61B 5/145

(54) **METHOD, MACHINE-LEARNING MODEL, DETECTING DEVICE, HOME SLEEP APNEA TEST DEVICE, COMPUTER PROGRAM PRODUCT, AND A COMPUTER-READABLE STORAGE MEDIUM**

(71) Applicant: Koninklijke Philips N.V., 5656 AG Eindhoven (NL)
(72) Inventor: ROSS, Marco, Eindhoven (NL); CERNY, Andreas, Eindhoven (NL); ANDERER, Peter Anderer, 5656AG Eindhoven (NL); DOS SANTOS DA FONSECA, Pedro Miguel Ferreira, Eindhoven (NL); SHAW, Edmund Arnliot, Eindhoven (NL); VASKO, Raymond, Eindhoven (NL)
(74) Representative: Philips Intellectual Property & Standards

(57) **Abstract**

There is provided a method for detecting sleep disordered breathing (SDB-) events. The method comprises receiving an arousal signal representative of an occurrence of an autonomic arousal; receiving an SDB-signal representative of an occurrence of an SDB-event; and generating a confirmation signal representing confirming that the SDB-signal is representative of the occurrence of the SDB-event based on the arousal signal. Further there is provided a detecting device for detecting SDB-events. The detecting device comprises a sensor system; and a processing unit connected to the sensor system. The sensor system is configured to generate an arousal signal and is configured to generate an SDB-signal. The arousal signal is representative of an occurrence of an autonomic arousal. The SDB-signal is representative of an occurrence of an SDB-event. The processing unit is configured to perform the method for detecting sleep disordered breathing events.

## Description

### FIELD OF THE INVENTION

The invention relates a method for detecting sleep disordered breathing (SDB-) events. Further, the invention relates to a machine-learning model for generating an arousal signal for use in the method. Further, the invention relates to a detecting device for detecting SDB-events comprising a processing unit to perform the method and/or comprising the machine-learning model. Further, the invention relates to a home sleep apnea test device comprising the detecting device. Further, the invention relates to a computer program product and a computer-readable storage medium.

### BACKGROUND OF THE INVENTION

In 2012, the American Academy of Sleep Medicine (AASM) revised the recommended scoring rule for hypopneas in polysomnography (PSG) recordings of adults: a respiratory event shall be scored as hypopnea if there is a 30% reduction in peak signal excursions from the pre-event baseline in the respiratory flow signal for at least 10 seconds and there is a ≥3% oxygen desaturation from pre-event baseline or the event is associated with an arousal. In contrast with the rules of previous versions of the AASM scoring manual, where the recommendation for hypopnea scoring could be met with only respiratory flow and oxygen desaturation signals, the updated manuals require the use of neurological signals to be able to score arousals. This limits the applicability of the rule to PSG recordings because neurological signals are typically not collected during home sleep apnea tests (HSATs). In fact, this limitation was addressed in version 22 of the AASM scoring manual with the addition of a new section focusing on HSAT rules for adults. In this new section, the AASM scoring manual recommends that a respiratory event shall be scored as hypopnea if there is a 30% reduction in peak signal excursions from the pre-event baseline in the respiratory flow signal for at least 10 seconds and there is a ≥3% oxygen desaturation from pre-event baseline.

Studies comparing HSATs to simultaneously recorded PSGs suggest that these polygraphic devices can achieve good performance for the categorization of SDB severity, especially when compared to the interscorer variability amongst manual experts scoring PSG. However, HSATs might underestimate the severity of mild and moderate SDB, which may impact the subsequent treatment plan.

### SUMMARY OF THE INVENTION

Known HSATs have two shortcomings. The first shortcoming is that HSAT-devices are not able to accurately detect sleep, because they do not include the recording of neurological signals. As a result they cannot provide a measure of total sleep time, which is required to calculate the apnea-hypopnea index (AHI). AHI is a clinically relevant measure of the presence and severity of OSA. Further, they cannot exclude false positive detections (due to natural, but otherwise OSA-unrelated characteristics of breathing) that occur during wake periods. The second shortcoming is that the known HSATs are not able to detect arousals, because they do not include the recording of neurological signals which are typically required to score these cortical events.

It is an objective of the invention to provide a method to detect SDB-events with improved accuracy without the need of neurological signals.

According to a first specific aspect, there is provided a method for detecting sleep disordered breathing (SDB-) events, comprising receiving an arousal signal representative of an occurrence of an autonomic arousal; receiving an SDB-signal representative of an occurrence of an SDB-event; and generating a confirmation signal representing confirming that the SDB-signal is representative of the occurrence of the SDB-event based on the arousal signal.

According to the first aspect, the method uses the arousal signal with is representative of the occurrence of an autonomic arousal. The occurrence of an autonomic arousal is detectable with various types of cost-effective and unobtrusive sensors that can be used at home. In comparison, detecting a cortical arousal requires a measurement with an EEG, which needs to be set up by an expert professional in a sleep center or hospital. The inventors have discovered that using a combination of the arousal signal and the SDB-signal provides an accurate indicated on whether the SDB-signal truly represents an SDB-event. By generating the confirmation signal, the method uses both the SDB-signal and the arousal signal to confirm whether the SDB-signal truly represents an SDB-event. For example, when the arousal signal indicates that the autonomic arousal occurs simultaneously with or within a few second from the SDB-event, the method confirms that the SDB-signal truly represents the occurrence of an SDB-event. For example, in case the SDB-signal indicates an SDB-event, while the arousal signal indicates no arousal, the method determines that the SDB-signal wrongly indicated an SDB-event. This way, the occurrence of SDB-events is accurately determined. As a result, the severity of SDB is determined more accurately.

The arousal signal is for example a signal from a cardiac sensor or from a respiratory sensor, or from a motion sensor. The cardiac sensor is, for example, an electrocardiogram (ECG) sensor, a photoplethysmogram (PPG-)sensor or a ballistocardiograph (BCG-) sensor or a seismocardiogram (SCG-) sensor. The respiratory sensor is, for example, an airflow sensor, or a pressure sensor, or an acceleration sensor configured to detect a breathing of a subject. The arousal signal is, for example, generated directly by a sensor. In another example, the arousal signal is generated by a preprocessing unit. The preprocessing unit is configured to receive a sensor signal from a single sensor or to receive sensor signals from multiple sensors. Based on the at least one the sensor signal, the preprocessing unit generates the arousal signal. For example, the preprocessing unit is configured to perform data processing on the sensor signal.

The SDB-signal is a signal representing an SDB-event such as an apnea-event or a hypopnea event or any other abnormal breathing event or any other breathing difficulty during sleep of a subject. The SDB-signal is, for example, generated by a respiratory sensor. The respiratory sensor is, for example, an airflow sensor, or a pressure sensor, or an acceleration sensor configured to detect a breathing of a subject. The SDB-signal is, for example, generated by a microphone configured to detect sound of breathing, or snoring, or any noise associated with an SDB-event, such as gasping, snorting or choking. The SDB-signal is, for example, generated directly by a sensor. In another example, the SDB-signal is generated by a preprocessing unit. The preprocessing unit is configured to receive a sensor signal from a single sensor or to receive sensor signals from multiple sensors. Based on the at least one the sensor signal, the preprocessing unit generates the SDB-signal. For example, the preprocessing unit is configured to perform data processing on the sensor signal. For example, a sensor system provided a plurality of sensor signals. The preprocessing unit is configured to generate the arousal signal based on a first subset of the plurality of sensor signals. The preprocessing unit is configured to generate the SDB-signal based on a second subset of the plurality of sensor signals. The first subset overlaps with the second subset, or the first subset does not overlap with the second subset.

The confirmation signal is generated based on the SDB-signal and the arousal signal. For example, the confirmation signal is based on a timing between the SDB-signal and the arousal signal. For example, the SDB-signal and the arousal signal indicate a timing relative to each other within 10 seconds or within 5 seconds. For example, the confirmation signal is based on an order of the SDB-signal and the arousal signal, such as that the SDB-signal is followed by the arousal signal. For example, the confirmation signal is based on a duration of the arousal signal.

In an embodiment, the method comprises generating a further confirmation signal representing confirming that the autonomic arousal is caused by the SDB-event based on the arousal signal and the SDB-signal.

According to this embodiment, the method not only confirms whether an SDB-event occurred, the method also confirms whether the arousal signal represents an autonomic arousal caused by the SDB-event. There are other causes for arousals than an SDB-event, such as noise in the environment of the sleeping subject, pain, or body movements. There are also natural arousals which serve as a natural interruption of deeper sleep stages. Especially in case the SDB-event is a hypopnea event, the further confirmation signal helps to indicate that the hypopnea caused the arousal. By determining whether the arousal was caused by the SDB-event, more insight is created on the severity of the SDB-event. This way, the AHI is determined more accurately.

In an embodiment, the arousal signal is based on at least one of a cardiac signal generated by a cardiac sensor and a respiratory signal generated by a respiratory sensor. The cardiac signal represents a cardiac parameter. The respiratory signal represents a respiratory parameter other than respiratory flow and SpO2. The SDB-signal is based on the respiratory signal.

The most direct way of determining an SDB-event, is by measuring the respiratory flow and the oxygen level in the blood via SpO2. In case of an SDB-event, the respiratory flow is reduced or blocked, and the oxygen level goes down significantly. However, sensors to measure respiratory flow and SpO2 are not commonly available for testing or treating SDB's at home. Also, many types of sensors to measure SpO2 are unsuited or too uncomfortable to be used for an extensive time, such as more than a few nights. The method is especially suited for use with a surrogate sensor, which is a respiratory sensor that measures a respiratory parameter other than respiratory flow and SpO2. The surrogate sensor measures, for example, thoracic case expansion, acceleration of the subject, or movement of the chest due to breathing. The method is especially suited for using a surrogate sensor, such as a bed sensor or a doppler sensor for measuring the respiratory parameter. Various wearable sensors are available, such as a chest belt. The surrogate sensor is, for example, cheaper and less intrusive. By combining the respiratory signal with the cardiac signal from the cardiac sensor, it is possible to obtain an improved accuracy in determining SDB-events.

In an embodiment, the method comprises receiving a sleep stage signal representative of a sleep stage of the subject; calculating a total sleep time of the user based on the sleep stage signal; and determining an apnea-hypopnea index (AHI) based on the SDB-signal, the confirmation signal and the total sleep time.

According to this embodiment, the sleep stage signal indicates in which sleep stage the subject is. There are four sleep stages: N1, N2, N3 and REM. There is one Wake state. Sleep stages N1 and N2 are light sleep stages. N3 is a deep sleep stage which is also known as slow-wave sleep. REM is a sleep stage with rapid eye movement and muscle atonia. The sleep stage signal indicates in case that the subject in not in one of the four sleep stages that the subject is awake. Sleep stages are characterized by various parameters, such as body temperature, muscle tone or lack thereof, eye movement, heart rate and heart rate variability. A sensor that is configured to detect one or more of these parameters generates the sleep staging signal to indicate in which sleep stage the subject is. For example, the sensor is a cardiac sensor, or a motion sensor, or a temperature sensor, or a respiratory sensor. For example, a preprocessor generates the sleep stage signal based on input from one or more sensors. For example, the preprocessor uses a signal from a sensor to generate two or more of the arousal signal, the SDB-signal and the sleep stage signal. By using the information about the sleep stage, the total sleep time can be accurately determined. As a result, the AHI is more accurately determined, providing a better insight on the severity of the SDB of the subject.

In a second aspect of the invention, there is provided a machine-learning model for generating an arousal signal for use in the method according to the first aspect of the invention. The machine-learning model comprises an arousal detection module and at least one of a cardiac feature extraction module and the respiratory feature extraction module. In an embodiment, the machine-learning model comprises the arousal detection module and both the cardiac feature extraction module and the respiratory feature extraction module. The cardiac feature extraction module is configured to generate an estimated cardiac parameter based on a cardiac signal representative of a cardiac parameter of a subject. The respiratory feature extraction module is configured to generate an estimated respiratory parameter based on a respiratory signal representative of a respiratory parameter of the subject. The arousal detection module is configured to generate an estimated arousal probability based on at least one of the estimated cardiac parameter and the respiratory parameter. The machine-learning model is configured to generate the arousal signal based on the estimated arousal probability. In an embodiment, the arousal detection module is configured to generate the estimated arousal probability based on both the estimated cardiac parameter and the respiratory parameter.

In an embodiment, the at least one of the cardiac feature extraction module, the respiratory feature extraction module and the arousal detection module comprises at least one residual convolutional network block. The at least one residual convolutional network block comprises a stack of at least two one-dimensional convolutions, the at least two one-dimensional convolutions having an exponentially increasing dilation rate, and at least one skip connection.

In an embodiment, the machine-learning model comprises a dense layer configured to receive an output from the stack of at least two one-dimensional convolutions.

In an embodiment, the machine-learning model comprises both the cardiac feature extraction model and the respiratory feature extraction module. Each of the cardiac feature extraction module, the respiratory feature extraction module and the arousal detection module comprises a residual convolutional network block. Each of the residual convolutional network blocks comprises a stack of at least two one-dimensional convolutions, the at least two one-dimensional convolutions having an exponentially increasing dilation rate, and at least one skip connection.

In an embodiment, the cardiac parameter comprises an estimation of the instant heart rate.

In an embodiment, the machine learning model has been trained by at least one of training the cardiac feature extraction module by deriving an instant heart rate signal from reference ECG data obtained in parallel to the cardiac signal; and training the cardiac feature extraction module, the respiratory feature model and the arousal detection module end-to-end using cortical arousal, derived from reference EEG data obtained in parallel to the cardiac signal and the respiratory signal, as a target.

In a third aspect of the invention, there is provided a detecting device for detecting SDB-events, comprising a sensor system and a processing unit connected to the sensor system. The sensor system is configured to generate an arousal signal and is configured to generate an SDB-signal. The arousal signal is representative of an occurrence of an autonomic arousal. The SDB-signal is representative of an occurrence of an SDB-event. The processing unit is configured to perform the method according to any one of claims 1-4.

In an embodiment, the sensor system comprises a first sensor and a second sensor. The first sensor is configured to measure a cardiac parameter of the subject. The second sensor is configured to measure a respiratory parameter of the subject other than respiratory flow and SpO2. The sensor system is configured to generate the arousal signal based on the cardiac parameter and the respiratory parameter.

In an embodiment, the detecting device comprises the machine-learning model of any one of claims 5-10.

In a fourth aspect of the invention, there is provided a home sleep apnea test device comprising the detecting device according to the third aspect of the invention.

In a fifth aspect of the invention, there is provided a computer program product, comprising instructions which, when executed by a processing unit, cause the processing unit to carry out at least one of the method of the first aspect of the invention and the machine-learning model of the second aspect of the invention.

In a sixth aspect of the invention, there is provided a computer-readable storage medium comprising at least one of instructions which, when executed by a processing unit, cause the processing unit to carry out the method of the first aspect of the invention and the machine-learning model of the second aspect of the invention.

### BRIEF DESCRIPTION OF THE DRAWINGS

Exemplary embodiments will now be described, by way of example only, with reference to the following Figures, in which:
FIG. 1 depicts an architecture overview of the autonomic arousal detection model according to an embodiment of the invention;
FIG. 2 depicts a comparison of the autonomic arousal index derived from home sleep apnea tests according to an embodiment of the invention to the arousal index from polysomnography;
FIG. 3 depicts a comparison of respiratory event indices derived from home sleep apnea tests to the apnea-hypopnea index derived from polysomnography;
FIG. 4 depicts a detecting device according to an embodiment of the invention.

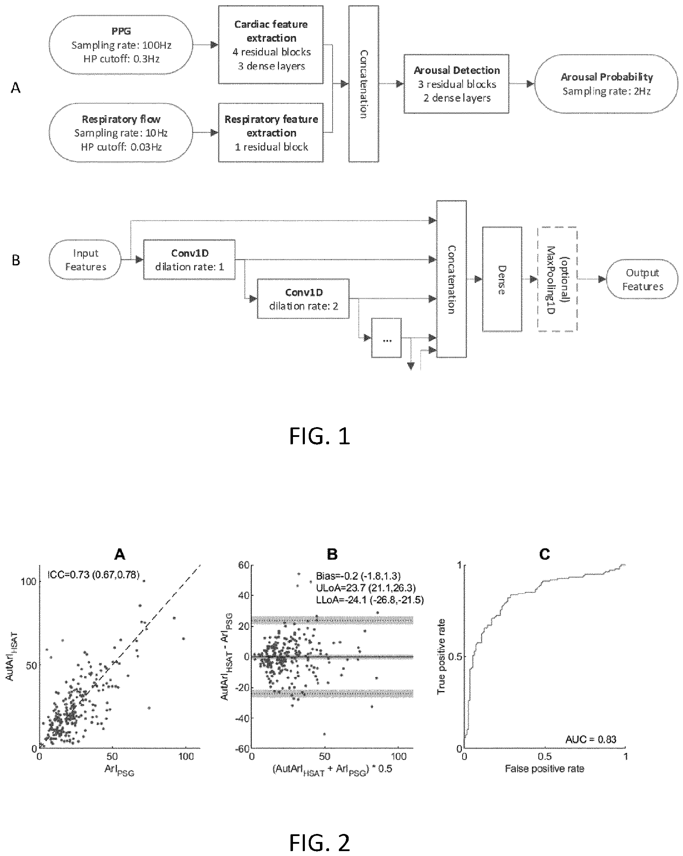

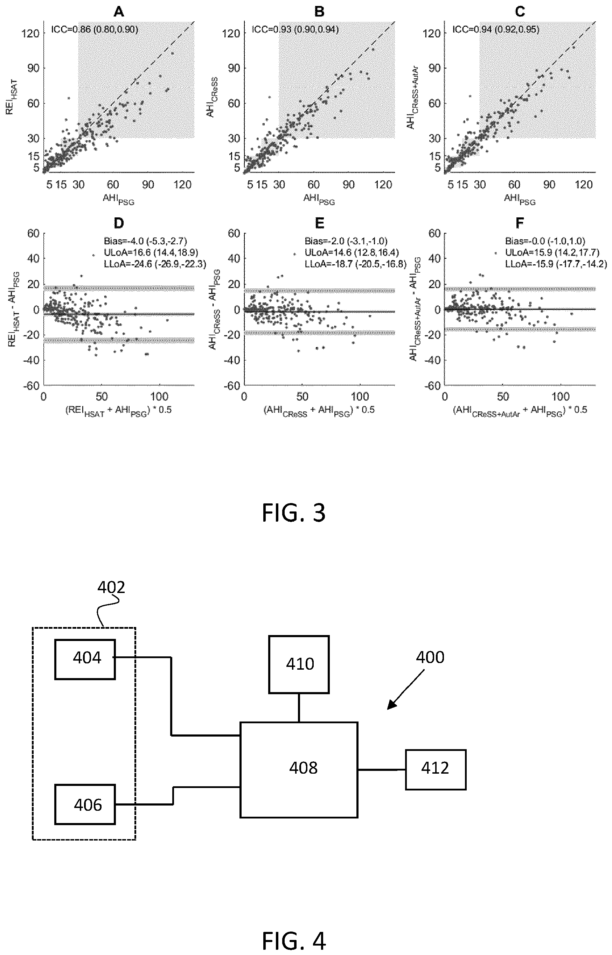

FIGS 2 and 3 are Figures in grey scale and are based on color drawings. The text below refers to the color in the original drawings.

### DETAILED DESCRIPTION OF EMBODIMENTS

The effect of autonomic arousal detection and cardio-respiratory sleep staging on the accuracy of home sleep apnea tests.

The manuscript reports on data from the clinical trial "Comparison of SomnaPatch with Polysomnography in Sleep Disordered Breathing" registered at clinicaltrials.gov with identification number NCT02034175 and accessible at https://clinicaltrials.gov/ct2/show/NCT02034175. References to literature are indicated with numbers in superscript. All references are incorporated by reference. Abstract

The objective of this study was to assess the impact of providing surrogate sleep and arousal information derived from autonomic nervous system activity on the diagnostic accuracy of home sleep apnea tests (HSATs), as compared to the currently recommended AASM scoring rules for respiratory events for polysomnography in adults.

We used the Somnolyzer auto-scoring algorithm to detect respiratory events, cortical arousals, and sleep stages in PSGs, and respiratory events and cardio-respiratory sleep staging (CReSS) in HSATs. We enhanced the Somnolyzer algorithm with an additional convolutional neural network to detect autonomic arousals in HSATs and performed a four-fold cross validation to compare the diagnostic performance of three different HSAT-derived surrogates for the apnea-hypopnea index based on monitoring time, CReSS-derived total sleep time, and additionally including autonomic arousal information for the scoring of hypopneas.

The intraclass correlation coefficient between the autonomic and the cortical arousal index, determined with the proposed method, was 0.73. Compared with using only monitoring time, sleep and arousal information led to an improvement in classification accuracy for no, mild, moderate, and severe sleep disordered breathing (SDB), from 70.2% (Cohen's κ=0.58) to 80.4% (κ=0.72) with a significant reduction of patients where the severity was underestimated from 18.8% to 7.3%.

We conclude that estimating sleep and arousal information from autonomic nervous system activity can improve the diagnostic sensitivity of HSATs while significantly reducing the risk of underestimating SDB severity without compromising specificity.

### Brief Summary

Most used home sleep apnea test devices do not provide sleep or arousal information. Therefore, the severity of sleep disordered breathing might be underestimated due to an overestimation of the total sleep time and an underestimation of the number of hypopneas that do not lead to oxygen desaturations but are associated with arousals.

In this study, we assessed the impact of using sleep and arousal information automatically derived from autonomic nervous system activity to improve the diagnostic accuracy of home sleep apnea tests. We show that in comparison with using only monitoring time, the inclusion of these surrogate sleep parameters leads to a significant improvement in severity classification agreement and a significant decrease in severity underestimation.

### Introduction

In 2012, the American Academy of Sleep Medicine (AASM) revised the recommended scoring rule for hypopneas in PSG recordings of adults: a respiratory event shall be scored as hypopnea if there is a 30% reduction in peak signal excursions from the pre-event baseline in the respiratory flow signal for at least 10 seconds and there is a ≥3% oxygen desaturation from pre-event baseline or the event is associated with an arousal.¹ In contrast with the rules of previous versions of the AASM scoring manual, where the recommendation for hypopnea scoring could be met with only respiratory flow and oxygen desaturation signals, the updated manuals require the use of neurological signals to be able to score arousals. This limits the applicability of the rule to PSG recordings because neurological signals are typically not collected during home sleep apnea tests (HSATs). In fact, this limitation (amongst others) was addressed in version 22 of the AASM scoring manual with the addition of a new section focusing on HSAT rules for adults.² In this section, the manual recommends that a respiratory event shall be scored as hypopnea if there is a 30% reduction in peak signal excursions from the pre-event baseline in the respiratory flow signal for at least 10 seconds and there is a ≥3% oxygen desaturation from pre-event baseline. Only in case sleep is recorded, a hypopnea can then be confirmed also by an associated arousal.

Historically, different hypopnea scoring criteria have been defined, requiring flow amplitude reductions of 30 or 50 percent, confirmation by 3% or 4% relative desaturations, confirmation with or without arousals, and sometimes even without any additional confirmation if the amplitude reduction was at least 50 percent.^{1,3,4} The impact of applying different hypopnea scoring rules on the diagnostic outcome has been studied in previous work, suggesting that using arousal events for the scoring hypopneas facilitates the identification and treatment of a wider spectrum of symptomatic patients without a significant loss of scoring reliability.⁵⁻⁷

Studies comparing HSATs to simultaneously recorded PSGs suggest that these polygraphic devices can achieve good performance for the categorization of SDB severity, especially when compared to the interscorer variability amongst manual experts scoring PSG. A systematic review of 59 studies evaluating the diagnostic accuracy of level 3 portable sleep tests compared to level 1 polysomnography concluded that HSATs are an appropriate diagnostic tool for adults with a high pretest probability of moderate to severe obstructive sleep apnea (OSA).⁸ However, they also highlight that the severity of mild and moderate SDB might be underestimated, which may impact the subsequent treatment plan.⁹⁻¹¹

Recently, artificial intelligence-based classifiers achieved remarkable results in classifying sleep from cardio-respiratory signals like photoplethysmography (PPG) and respiratory effort/flow or peripheral-arterial tonometry (PAT), effectively enabling the scoring of sleep in HSAT recordings without neurological signals.¹²⁻¹⁸ Although these algorithms do not reach the accuracy of sleep staging using neurological signals and usually do not allow the discrimination between N1 and N2 sleep, it has been shown that they can provide accurate estimates of the total sleep time as well as of REM sleep periods. Given the crucial role of total sleep time as a denominator in the estimation of indices describing the frequency of respiratory disturbances per hour of sleep, it thus follows that these surrogate estimations help improve the diagnostic sensitivity of HSATs for detecting sleep disordered breathing (SDB) and can even enable the detection of REM-related obstructive sleep apnea (OSA).^{12,19} Similarly, advances were made in the detection of autonomic arousals in HSATs as a surrogate for cortical arousals. Using PAT, or electrocardiography, they achieve good agreement in the detection of arousals even in the absence of the neurological signals traditionally used to score them.²⁰⁻²² Devices using autonomic arousals or sympathetic activation events to aid the detection of respiratory events are already available.¹¹

We hypothesize that an accurate estimation of total sleep time combined with an accurate detection of autonomic arousals used to confirm hypopneas - even if they are not associated with an oxygen desaturation - will significantly improve the diagnostic accuracy of HSATs.

In this study we evaluated the effect of applying the recommended scoring rules for respiratory events as defined for PSG but based on signals acquired with HSATs instead. Towards this end, we used a previously developed cardiorespiratory sleep staging to obtain a surrogate measure of total sleep time, and developed an artificial neural network to detect autonomic arousals. We then assessed the performance impact on apnea-hypopnea index (AHI) estimation accuracy by comparing it against AHI estimated based on full PSG.

### Methods

### Datasets

To analyze the effect of autonomic arousal detection on the diagnostic accuracy of HSATs, acquisitions from two databases were combined to train an artificial neural network to detect autonomic arousals.

The first database, called Somnoval, consisted of 97 routine in-lab PSG recordings of patients referred to three different sleep laboratories that had been used to validate the Somnolyzer auto-scoring sytem.²³ Each PSG recording was scored independently by four registered polysomnographic technologists (RPSGTs) according to the version 1.0 of the AASM scoring guidelines including sleep stages, arousals, and respiratory events. The data consisted of approximately one third diagnostic studies, one third positive airway pressure (PAP) titration studies, and one third split nights where the first portion of the recording contains a diagnostic montage confirming obstructive sleep apnea (OSA) while the second portion of the night is used for PAP titration. The study protocol was approved by the institutional review board of each clinical site. We artificially created parallel HSAT recordings by copying a limited set of channels that would typically be available in a HSAT (respiratory flow, thoracic effort, SpO2, snoring, pulse rate, body position, raw PPG). Only one flow signal was retained when creating the reduced (HSAT) versions of the PSG studies: the nasal pressure flow in diagnostic nights and for the first portion of split nights, and the treatment device flow during titration nights and second portions of split nights.

The second database, called Somnapatch, consisted of 190 acquisitions with full PSG and simultaneously recorded HSAT using a shared nasal canula. We used the pressure flow signals sampled at 100Hz for exact time-alignment of the parallel recordings, correcting for clock shift and clock drift using cross-correlation between the two recordings in moving windows. We excluded all recordings where the quality of one or both pressure flow signals was too low to reliably determine the clock shift and clock drift for the entire recording. Twelve acquisitions were excluded due to missing data or bad data quality and another thirty recordings were excluded because the time-alignment between the PSG and the HSAT recordings was not reliably possible, resulting in a total number of 148 recordings from this database that were used in the present study. Informed consent was obtained during the first visit of each participant, prior to the monitoring night.

Table 1 summarizes the demographic information and the distribution of SDB severity for the two databases.

### Detection of autonomic arousals using artificial intelligence

A deep convolutional neural network was developed to detect autonomic arousals using the raw PPG signal and the respiratory flow signal as inputs. The model consists of three modules that are illustrated in FIG 1A. All parts of the model use residual convolutional network blocks as illustrated in FIG 1B, which successively increase the feature complexity while reducing temporal resolution.

FIG 1A provides an overview over the architecture of the autonomic arousal detection model developed in the present study. There are two feature extraction modules that extract cardiac features from the photoplethysmography signal and respiratory features from the respiratory flow signal. The cardiac and respiratory features are combined and used as input for the final arousal detection module which generates an arousal probability sampled at 2Hz. All parts of the model use residual convolutional network blocks as illustrated in FIG 1B to successively increase the feature complexity while reducing temporal resolution. FIG 1B illustrates the architecture of the residual convolutional blocks that were used throughout the model.

Inspired by the encoder portion of the atrous spatial pyramid pooling scheme presented by Chen et al.,²⁴ stacked one-dimensional convolutions with small kernels, exponentially increasing dilation rates, and skip connections can extract complex features from large temporal contexts with a relatively small set of parameters. While in our model we always used three convolutional layers per residual block, an arbitrary number of convolutional layers is theoretically possible. Finally, a dense layer is used to create a more compact representation of the features and an optional maximum pooling layer reduces the temporal resolution.

Using four residual convolutional blocks, the first module consists of a cardiac feature extraction module, developed to estimate instantaneous heart rate (IHR). In the present study we used PPG as input, resampled to 100Hz and pre-processed with a 0.3Hz high pass filter. This module was trained separately with an additional data augmentation step where inputs were temporally stretched and squeezed within a predefined range to cover a wide range of possible heart rates. The target for training this module consisted of IHR derived from parallelly recorded ECG data from the full PSG using the R-peak localization algorithm developed by Fonseca et al.²⁵ Although in the present study we used PPG, we deliberately chose to use IHR as the only cardiac feature in the arousal detection model in order for the final algorithm to be agnostic regarding the actual cardiac input signal, which can be, besides PPG, also ECG, or any other cardiac sensor modality.

The second module consists of a respiratory feature extraction block comprised of a single residual block, which uses as input respiratory flow resampled at 10Hz and high pass filtered with a cut-off frequency of 0.03Hz. Together with the cardiac feature extraction and the arousal detection modules, the entire model was trained end-to-end using cortical arousals as the target. During this second training phase, the parameters of the cardiac feature extraction module were fixed. The training targets were sequences sampled at 2Hz containing the scorings of cortical arousals according to the scoring rules defined in the AASM scoring manual version 2.626 from Registered Polysomnographic Technologists (RPSGTs) as well as the Somnolyzer auto-scoring system. Where multiple scorings were available, soft targets with probabilities between 0 and 1 were used.

The output of the model can be interpreted as a continuous arousal probability sampled at 2Hz. Consecutive sequences of output samples where the arousal probability exceeded a pre-defined threshold for at least 2 seconds were considered as part of (individual) autonomic arousal events.

The dataset contained only one recording per subject and was split into four folds containing 25% of the subjects of each database. Subject selection was performed using pseudo-random sampling without replacement, using a fixed seed to guarantee reproducibility. Four-fold cross-validation was performed by iteratively and sequentially combining three folds as training set - used to train the neural network - and evaluating its performance on the data of the remaining validation fold, which was never used in the same iteration to fit, tune or otherwise adjust any parameter of the model for that iteration. The threshold used to detect autonomic arousal events from the output probabilities was chosen individually for each of the four models (one per iteration) by balancing precision and recall for the detection of arousals on the respective training set.

### Scoring of sleep recordings

All recordings were analyzed according to the sleep and sleep disordered breathing scoring rules recommended in the AASM manual version 2.626 with the Somnolyzer auto-scoring system that has recently been validated and shown to be non-inferior to manual expert scoring.²⁷ In particular, hypopneas were confirmed by three percent relative oxygen desaturations and/or cortical arousals in full PSG recordings and by three percent relative oxygen desaturations and/or autonomic arousals in HSAT recordings. In full PSG recordings, sleep stages and arousals were derived from neurological signals using all frontal, central, occipital EEG, both EOG, and the chin EMG channels. In HSAT recordings, we used the clinically validated CReSS algorithm as published in Bakker et al.,¹² to infer sleep stages and total sleep time. Autonomic arousals were detected using our novel artificial neural network. Using cross-validation as described, autonomic arousals were always scored with the model that had been trained without any data from the current subject, i.e., when that subject was part of the validation fold.

To calculate the arousal index, we counted cortical and autonomic arousals if they started during sleep or within 15 seconds of a sleep epoch; the tolerance of 15 seconds was introduced to also capture arousals that lead to awakenings. For the calculation of respiratory event indices, we counted all apneas and hypopneas that overlapped with a sleep epoch (even if they started or ended in a wake epoch) and divided the number of respiratory events by the total sleep time in hours.

### Statistical analyses

To assess the agreement between the HSAT-derived autonomic arousal index (AutArI) as the number of autonomic arousals per hour of sleep as determined by CReSS and the arousal index (ArI) derived from the PSG, we generated scatter plots, calculated the intraclass correlation coefficient (ICC) of absolute agreement with its 95% confidence interval, generated Bland-Altman plots, and calculated the bias and levels of agreement with 95% confidence intervals. Furthermore, we replicated the receiver-operating characteristic (ROC) analysis used by Pillar et al. to assess the autonomic arousal index's performance in detecting a pathological ArI ≥ 20 by varying the threshold for the autonomic arousal index.^{20,28}Since the apnea-hypopnea index (AHI) is the most commonly used metric for assessing SDB severity, we also compared three different HSAT-derived surrogates for the AHI with the gold-standard derived from full PSG (AHIPSG). The first estimate corresponds to the traditionally reported respiratory event index (REIHSAT) which uses as denominator the entire monitoring or recording time and does not consider autonomic arousals for the confirmation of hypopneas. The respiratory event index was thus calculated as the number of apneas and hypopneas (confirmed by oxygen desaturations ≥ 3%) scored per hour of recording time. The second estimate (AHICReSS) uses sleep stages estimated by the CReSS algorithm to filter respiratory events that were scored during wakefulness and uses the accurate estimate of total sleep time derived from the cardio-respiratory sleep staging, and was calculated as the number of apneas and hypopneas (confirmed by oxygen desaturations ≥ 3%) scored during sleep, per hour of sleep as determined by CReSS. Finally, the third estimate (AHICReSS+AutAr) considers the CReSS-derived sleep-wake information and additionally uses autonomic arousals to score hypopneas. It was scored as the number of apneas and hypopneas (confirmed by oxygen desaturations ≥ 3% or autonomic arousals) scored during sleep per hour of sleep as determined by CReSS.

To compare the surrogate measures to the ground-truth AHIPSG, we generated scatter plots and calculated the intraclass correlation coefficient (ICC) of absolute agreement29 with 95% confidence intervals. Furthermore, we generated Bland-Altman plots30 and calculated the bias and levels of agreement with 95% confidence intervals. To demonstrate the effects of using different AHI surrogates, we provided confusion tables for the categorization of SDB into None (AHI < 5), Mild (5 ≤ AHI < 15), Moderate (15 < AHI ≤ 30), and Severe (AHI ≥ 30). We calculated accuracy, sensitivity, specificity, positive likelihood ratio (LR+), negative likelihood ratio (LR-), and the Cohen's κ for detecting an AHI greater than or equal to thresholds of 5, 15, or 30. Furthermore, we calculated the Cohen's κ, accuracy for all 4 classes combined and the fraction of subjects where SDB severity was under- or overestimated. We tested for statistically significant differences between the performance of the AHICReSS and the REI as well as between the AHICReSS+AutAR and the REI. Following the guidelines for statistical testing in clinical trials presented by Kishore and Mahajan, we used two-sided 95% confidence intervals and P-values to determine which metrics differed significantly.³¹ For binomial proportions, confidence intervals were estimated (accuracy, sensitivity, specificity, fraction of under- or overestimated SDB severity) using the Wilson Score interval with continuity correction.³² We used the method proposed by Simel et al. to estimate confidence intervals for likelihood ratios.³³ The formula for estimating confidence intervals for Cohen's κ coefficients was provided by Cohen himself.³⁴ Given an acceptable false detection rate of 5%, we applied the Benjamini-Yekutieli procedure to all tests regarding the AHICReSS as well as to all tests regarding the AHICReSS+AutAr to control the false discovery rate in multiple tests under arbitrary dependency.³⁵

### Results

FIG 2 compares the HSAT-derived autonomic arousal index (AutArI) to the gold-standard arousal index (ArI) derived from PSG. The Intra-class correlation coefficient was 0.73 with a 95% confidence interval of (0.67, 0.78) and a bias in the estimation of the arousal index of -0.2 events/hour with a 95% CI of (-1.8, 1.3). The levels of agreement and their respective 95% confidence intervals were -24.1 (-26.8, -21.5) and 23.7 (21.1, 26.3) events/hour. We assessed the autonomic arousal index's diagnostic ability for detecting an ArI ≥ 20 (134 out of the 245 subjects in the database) with a receiver-operating characteristic (ROC) curve, achieving an area under the ROC curve of 0.83. FIG 3 illustrates the comparison of HSAT-derived surrogates to the gold standard AHIPSG for the combined dataset (97 Somnoval plus 148 Somnapatch studies). Using the most common clinical HSAT practice of using the REI (REIHSAT) resulted in an intra-class correlation coefficient of 0.86 with a 95% confidence interval (CI) of (0.80, 0.90) and a mean difference (bias) of -4.0 events/hour with a 95% CI of (-5.3, -2.7). Using cardio-respiratory staging to estimate total sleep time and to remove false positive events during wakefulness (AHICReSS), improved the ICC to 0.93 with a 95% CI of (0.90, 0.94) and the bias to -2.0 events/hour with a 95% CI of (-3.1, -1.0). Finally, by additionally scoring autonomic arousals and confirming hypopneas associated with them (AHICReSS+AutAr), the ICC was improved to 0.94 with a 95% CI of (0.92, 0.95) and the bias was reduced to 0.0 with a 95% CI of (-1.0, 1.0). With each improvement of the index estimation, also the levels of agreement for the differences come closer to the bias. Furthermore, the Bland-Altman plot in panel D comparing the REIHSAT to the AHIPSG clearly shows an underestimation proportional to the severity which is much less pronounced or even absent in panels E and F comparing the AHICReSS and the AHICReSS+AutAr to the AHIPSG.

In FIG 3, panels A-C show scatter plots of the respiratory event index as the number of apneas and hypopneas events per hour of monitoring time (REIHSAT), the number of apneas and hypopneas per hour of total sleep time derived from cardio-respiratory signals (AHICReSS), and the number of apneas and hypopneas per hour of total sleep time derived from cardio-respiratory signals where autonomic arousals were used to confirm hypopneas (AHICReSS+AutAr) as compared to the AHIPSG and provide the respective intraclass correlation coefficients of absolute agreement (ICC) and their respective 95% confidence intervals in brackets. The green background color indicates areas where both, the surrogate and the gold-standard measurement would yield the same severity classification using thresholds of 5, 15, and 30. The bottom row (panels D-F) shows Bland-Altman plots to illustrate differences between the surrogate measurements REIHSAT, AHICReSS, and the AHICReSS+AutAr and the gold-standard AHIPSG and includes the bias and levels of agreement for the surrogate measures with their respective 95% confidence intervals in brackets. One outlier was identified and highlighted in red (see the discussion section for information about the outlier).

F 2 shows in panel A a scatter plot to compare the autonomic arousal index (AutArIHSAT) to the cortical arousal index (ArIPSG) and the intraclass correlation coefficient of absolute agreement with a 95% confidence interval in brackets. Panel B illustrates differences between the AutArIHSAT and the ArIPSG in a Bland-Altman plot and provides bias and levels of agreement together with their respective 95% confidence intervals in brackets. Panel C shows the autonomic arousal index's receiver operating characteristic (ROC) curve and the area under the curve for detecting an arousal index ≥ 20. We identified four outliers and highlighted them in red (see the discussion section for information about the outliers).

Table 2 compares the three AHI surrogates with respect to their ability to correctly assess the severity of sleep disordered breathing. We generated confusions tables for the three AHI estimations comparing the resulting severity classification into no, mild, moderate and severe SDB using thresholds of 5, 15, and 30 to the ground-truth derived from the full PSG.³⁶ The diagonals of the matrices (blue) show the number of correct classifications, while underestimations of SDB severity are found above the diagonal, and overestimations are located below the diagonal. Overall, using the REIHSAT resulted in the correct severity classification of 172 subjects (70.2%, κ=0.58) while severity was underestimated in 46 subjects (18.8%) and overestimated in 27 subjects (11.0%). After applying cardio-respiratory sleep staging and using the AHICReSS to estimate severity, 189 subjects (77.1%, κ=0.67) subjects were classified correctly, and the severity was underestimated in 32 subjects (13.1%) and overestimated in 24 subjects (9.8%). Finally, when hypopneas were scored with autonomic arousals and the AHICReSS+AutAr was used to assess SDB severity, the number of correctly classified subjects increased to 197 (80.4%, κ=0.72) and the number of under-diagnosed subjects was further reduced to 18 (7.3%). The number of subjects with overestimated SDB severity slightly increased to 30 (12.2%). It is worth mentioning that for all three AHI surrogates, most misclassifications were between adjacent severity categories. The number of subjects where the severity was under- or overestimated by more than one severity category was 4 (1.6%), 0, and 1 (0.4%) when using the REI, AHICReSS and AHICReSS+AutAr, respectively.

Table 3 and Table 4 compare the diagnostic performance of AHICReSS and AHICReSS+AutAr to the REI. The Benjamini-Yekutieli procedure was applied to all statistical tests comparing the AHICReSS to the REI, resulting in a threshold for P-values of 0.0012 that yields two significant comparisons. For tests comparing the AHICReSS+AutAr to the REI, the procedure resulted in a threshold for P-values of 0.0062, yielding 11 significant comparisons. Statistically significant differences are highlighted.

Table 3 summarizes performance measures and provides 95% confidence intervals for the binary classification tasks of determining an AHI greater or equal than the diagnostic thresholds of 5, 15, and 30 and presents overall performance statistics with 95% confidence intervals for the four-class classification problem into none, mild, moderate, and severe SDB. For the detection of an AHI ≥ 5, the introduction of CReSS lead to statistically significant, but clinically irrelevant changes, in the sensitivity and in the negative likelihood ratio: the sensitivity was reduced from 1.0 to 0.99 and the negative likelihood ratio increased from 0.0 to 0.01. For an AHI ≥ 15, the combination of cardio-respiratory sleep staging and autonomic arousal detection yielded statistically significant improvements for the accuracy which increased from 0.873 to 0.931, the sensitivity which increased from 0.882 to 0.947 and the Cohen's κ which increased from 0.712 to 0.837. The specificity and the positive and negative likelihood ratios improved as well, but these changes were not statistically significant. For the detection of an AHI ≥ 30, the introduction of CReSS and the hypopnea scoring using autonomic arousals lead to statistically significant improvements in sensitivity which increased from 0.755 to 0.934, the negative likelihood ratio that decreased from 0.256 to 0.073 and the Cohen's κ which increased from 0.728 to 0.835. The accuracy improved as well, while the specificity and the positive likelihood ratio were both reduced. However, these changes were not statistically significant.

Table 4 present performance metrics for the four-class classification problem. By adding sleep and arousal information derived from autonomic features, significant improvements could be achieved regarding the overall accuracy with an improvement from 0.702 to 0.804, the Cohen's κ which increased from 0.58 to 0.716, as well as the fraction of subjects where SDB severity was underestimated which was reduced from 0.188 to 0.073. There were no significant changes in the fraction of subjects where SDB was overestimated.

### Discussion

The AHI is the primary measure for quantifying SDB severity in clinical practice. Diagnostic thresholds of 5, 15, and 30 are recommended to discriminate between no, mild, moderate, and severe OSA.³⁶ In full PSGs, the AHI can be considered to be a very robust measure with high inter-scorer agreement, with reported ICCs across independent manual scorings between 0.84 and 1.00 for the alternative hypopnea scoring rules (confirmation by a ≥ 4% desaturation only) and between 0.84 and 0.93 for the recommended hypopnea scoring rules (confirmation by a ≥ 3% desaturation or an arousal), with most values well above 0.90 for both scoring rules.^{10,11,23,37-39} In this work, we focused on the recommended scoring rules where hypopneas shall be scored if there is a relative oxygen desaturation of at least 3% or if they are associated with an arousal. We found that applying the recommended scoring rules for hypopneas to HSATs using cardio-respiratory sleep staging and autonomic arousals increased the ICC of absolute agreement between the HSAT-derived surrogate and the gold standard of PSG-derived AHI to 0.94 with a 95% confidence interval of (0.92, 0.95) indicating excellent agreement that is in the same range as the inter-scorer agreement between manual scorers scoring gold-standard PSGs.²⁹ Using recommended hypopnea scoring rules, manual experts were reported to agree on the AHI with a bias of less than ±1.5 and a 95% limits of agreement of approximately +/- 18 to 22 events per hour of sleep when scoring full PSG studies.^{10,11} We observed a bias of -4 events/hour when using the REI, confirming our initial hypothesis that REI can, and often underestimates SDB severity. This bias could be eliminated completely by using CReSS to determine the total sleep time and by confirming hypopneas not only by desaturations but also by autonomic arousals, further narrowing the limits of agreement to less than ±16 events per hour of sleep. As expected, the agreement between the HSAT-derived REI and the PSG-derived AHI fell below typical inter-scorer reliability reported for the AHI. Adding sleep and arousal estimations from the autonomic nervous system raised the agreement to the same level as observed between manual scorers scoring full PSG recordings, 10,11 suggesting an improvement on diagnostic accuracy.

Regarding the binary classification at diagnostic thresholds of 5, 15 and 30, we expected an increase in sensitivity for all three thresholds, possibly at the cost of small losses in specificity, contributing to an overall increase in accuracy. Surprisingly, it was the specificity for detecting an AHI ≥ 5 that increased substantially from 53.3% to 73.3% when introducing CReSS-derived total sleep time in the AHI calculation. At first, this might seem counter-intuitive because the reduction of the denominator in the AHI calculation from monitoring time to total sleep time should increase the estimated AHI and therefore increase diagnostic sensitivity. A closer look at the scorings revealed that some of the false positives (REI ≥ 5 but AHI < 5) were caused by respiratory events detected during wakefulness, possibly caused by motion artifacts. CReSS excluded these respiratory disturbances during wakefulness, effectively increasing the specificity of the HSAT devices without any relevant loss in sensitivity. Although statistically significant, the reduction of sensitivity from 100% to 99% and the corresponding increase in the negative likelihood ratio from 0.0 to 0.01 are neglectable. For the task of detecting an AHI ≥ 15, which is often used as a threshold for reimbursement of treatment, the introduction of CReSS increased sensitivity and specificity, and therefore also the accuracy from 87.3% to 91.8%. Sensitivity and accuracy were increased further by confirming hypopneas with autonomic arousals such that the final accuracy was 93.1% with a sensitivity of 94.7% and a specificity of 89.3%. Again, sensitivity and specificity both clearly improved when adding the sleep and arousal information to the HSAT recordings. Only the combination of both resulted in statistically significant increases of accuracy, sensitivity, and the Cohen's κ coefficient. Finally, for an AHI ≥ 30, sensitivity increased greatly from 75.5% to 93.4% while the specificity remained above 90% and the accuracy improved from 86.9% to 91.8%. The combination of sleep and arousal information yielded statistically significant improvements over the use of the REI regarding the sensitivity, the negative likelihood ratio and the Cohen's κ coefficient.

Van Pee et al. reported accuracies and Cohen's κ values for the interscorer agreement of 93% (κ=0.69), 90% (κ=0.79), and 92% (κ=0.83) at diagnostic thresholds of 5, 15, and 30, respectively.¹⁰ From the confusion tables presented by Massie et al. we can easily derive very similar accuracies and Cohen's κ values for the interscorer agreement of 93% (κ=0.68), 91% (κ=0.81), and 89% (κ=0.76) for the respective diagnostic thresholds.¹¹ When comparing these values to our results, it became clear that the classification performance with REI was only comparable to the manual scoring of full PSGs for a diagnostic threshold of the AHI being greater or equal to 5. When adding sleep and arousal estimations from the autonomic nervous system to the HSAT analysis, the SDB classification performance at all three thresholds increased to the same level as observed between manual experts scoring full PSG recordings with accuracies and Cohen's κ values of 95.1% (κ=0.74) for detecting an AHI ≥ 5, 93.1% (κ=0.84) for an AHI ≥ 15, and 91.8% (κ=0.83) for an AHI ≥ 30. When classifying the SDB severity into four classes, Massie et al. reported an interscorer agreement on full PSGs of 81% with a Cohen's κ of 0.74, and Van Pee et al. reported an interscorer agreement of 77% with a Cohen's κ of 0.66.^{10,11} As can be seen in Table 4, using the REI as surrogate for the AHIPSG, we discovered an agreement of just 70.2% (κ=0.58) which clearly fell below the reported inter-scorer agreement. However, the agreement on severity subclassification increased to 77% (κ=0.67) by adding CReSS-derived sleep information and then further to 80.4% (κ=0.72) by adding autonomic arousal information to the HSAT's event scoring, boosting classification performance up to the level of interscorer agreement amongst manual experts scoring full PSG recordings. Using the combination of sleep and arousal information derived from cardio-respiratory signals the overall classification performance was significantly improved regarding the overall accuracy, Cohen's κ, and the percentage of participants where SDB was underestimated. At the same time, the increase of over-diagnosed patients from 11% to 12.2% was neither statistically significant (p=0.596) nor clinically relevant. These results confirm that using the AHICReSS+AutAr to assess SDB severity instead of the REI significantly improves the diagnostic sensitivity of HSAT devices, leading to much smaller likelihood of underestimating SDB severity.

In FIG 2, we identified one outlier where the AHI was severely overestimated by all three HSAT-derived estimates. The AHIPSG for this recording was 21.9 while HSAT-derived estimates varied between 64.3 and 66.0. A closer look at the raw data of this recording revealed that this recording contained numerous central apneas during epochs that contained rapid transitions between wake and sleep. The occurrence of sleep transition apnea may yield immediate hyperventilation leading to central apnea upon resumption of sleep.⁴⁰ This alternating pattern has been described as state instability in previous research. 41 In this example, most of these epochs had to be scored as wake because the EEG data indicated wakefulness for more than half of each 30-epoch. Consequently, most of these apneas were not considered for the calculation of the AHIPSG. This presents a more fundamental limitation of scoring sleep stages in 30-second epochs and could only be fully resolved by adopting, for example as discussed by Perslev et al., high-frequency sleep staging, especially for diagnosing a population experiencing sleep apnea.⁴² Due to the lack of sleep information, these events were included in the calculation of the REIHSAT. Somnolyzer's CReSS algorithm scored these transitional epochs as sleep, therefore this recording remained an outlier even when adding sleep and arousal estimations to the HSAT scoring. Regarding arousal detection, interscorer agreement for the number of arousals and the arousal index have been reported with intraclass correlation coefficients between 0.52 and 0.80 with outliers as low as 0.09 indicating only limited reliability.^{23,37-39,43} In an early attempt at detecting autonomic arousals from ECG, agreement between cortical and autonomic arousals has been reported with an ICC of 0.19.⁴⁴ More recently developed algorithms based on PAT or ECG inputs report Pearson's correlation coefficients between 0.58 and 0.84, depending on the OSA severity distribution of the patients.^{20,21} Olsen et al. report an event-by-event sensitivity of 0.63 and positive predictive value of 0.72.²² The ICC between the autonomic arousal index derived from our model using PPG and respiratory flow as inputs and the cortical arousal index derived from neurological signals in the PSGs was 0.73 with a 95% confidence interval of (0.67, 0.78) which clearly fell within the range of normal interscorer reliability of scoring cortical arousals in a full PSG and is similar to the performance reported for PAT devices detecting autonomic arousals. It is clear that autonomic arousals are not the same as cortical arousals and not every cortical arousal coincides with an autonomic arousal, and vice versa; especially periodic limb movements and respiratory events appear to be often associated with autonomic arousals without a corresponding cortical arousal.^{22,45,46} Nevertheless, the agreement between autonomic arousals and cortical arousals has been reported in a similar range as the interscorer agreement on scoring cortical arousals, which further supports the idea of using autonomic arousals as surrogate measurement for arousals in HSATs.

We identified four outliers when comparing the cortical arousal index to the autonomic arousal index and highlighted them in FIG 3. In one case a cortical arousal index of 75.0 was heavily underestimated, with an autonomic arousal index of 24.2. A review of the raw data revealed that this patient had severe cardiac arrhythmia which probably reduced the sensitivity of the autonomic arousal detection. Moreover, we identified three cases where relatively low cortical arousal indices of 5.3, 8.2, and 16.0 were overestimated with autonomic arousal indices of 59.2, 54.5, and 64.8. In two of these cases, the autonomic arousals were associated with respiratory events that were not associated with cortical arousals. The third case contained periodic leg movements that were associated with autonomic arousals but not with cortical arousals. Both phenomena have been described previously: Olsen et al. reported that 81% of autonomic arousals, not associated with a matching cortical arousal, could be related to respiratory events or leg movements,22 Pillar et al. reported that the agreement between the cortical and autonomic arousal index could be increased significantly when excluding patients with periodic leg movements from the comparison.²⁰ In our case, the ICC between the autonomic arousal index and the cortical arousal index increased from 0.73 (95%CI 0.67, 0.78) to 0.79 (95%CI 0.74, 0.84) when excluding these four outliers.

In conclusion, this study showed that adding sleep and arousal information derived from autonomic nervous system activity can greatly improve the diagnostic sensitivity of HSATs by significantly reducing the risk of underestimating SDB severity without any relevant decrease in specificity. Especially, the confirmation of hypopneas using autonomic arousals can raise the sensitivity to a level similar to that of a full PSG recording. Furthermore, the autonomic arousal index can serve as a viable surrogate of the cortical arousal index as a measure of sleep fragmentation.

The lack of visual scoring rules for autonomic arousals and sleep staging from cardio-respiratory signals or peripheral arterial tonometry implies that we must rely on computer-based algorithms to provide this information. Recently, advances in machine learning provided powerful tools for solving complex tasks in pattern recognition such as sleep staging and arousal detection. Since these algorithms typically operate as black boxes, validation studies with large datasets covering the whole spectrum of the population eligible for HSATs will be required to prove the safety and efficacy of the sleep and autonomic arousal scoring of individual devices and algorithms. These validation datasets should be completely unseen by the models both during training and internal validation and ideally, collected in different centers and scored by different (pools of) human experts.

FIG. 4 depicts a detecting device 400 for detecting SDB-events according to an embodiment of the invention. The detecting device 400 comprises a sensor system 402, a processing unit 408, a memory 410 and an output terminal 412. The sensor system 402 is configured to generate the arousal signal and is configured to generate the SDB-signal. The arousal signal is representative of an occurrence of an autonomic arousal. The SDB-signal is representative of an occurrence of an SDB-event. The processing unit 408 is configured to perform the method according to the invention.

The sensor system 402 comprises a first sensor 404 and a second sensor 406. The first sensor 404 is configured to measure a cardiac parameter of the subject. The second sensor 406 is configured to measure a respiratory parameter of the subject other than respiratory flow and SpO2. The sensor system 402 is configured to generate the arousal signal based on the cardiac parameter and the respiratory parameter. The memory 410 is a computer-readable storage medium having there on a computer program product. The computer program product comprises instructions which, when executed by the processing unit 408, cause the processing unit 408 to carry out at least one of the method and the machine-learning model as described above. The processing unit 408 provides an output signal to the output terminal 412. The output terminal 412 provides output information representing the detected sleep disordered breathing events.

### Abbreviations

- SDB: Sleep disordered breathing
- OSA: Obstructive sleep apnea
- PSG: Polysomnography
- HSAT: Home sleep apnea test
- ICC: Intraclass correlation coefficient
- PPG: Photoplethysmography
- ECG: Electrocardiography
- EEG: Electroencephalography
- EMG: Electromyography
- EOG: Electrooculography
- PAT: Peripheral arterial tonometry
- CReSS: Cardio-respiratory sleep staging
- REM: Rapid eye movement sleep
- AHI: Apnea-Hypopnea index
- REI: Respiratory event index
- ArI: (Cortical) arousal index
- AutArI: Autonomic arousal index
- AASM: American Academy of Sleep Medicine
- PAP: Positive airway pressure
- CPAP: Constant positive airway pressure
- RPSGT: Registered polysomnographic technologist

### References

1. Berry RB BR, Gamaldo CE, Harding SM, Marcus CL and Vaughn BV for the American Academy of Sleep Medicine. The AASM Manual for the Scoring of Sleep and Associated Events: Rules, Terminology and Technical Specifications, Version 2.0. www.aasmnet.org, Darien, Illinois: American Academy of Sleep Medicine; 2012.
2. Berry RB BR, Gamaldo CE, Harding SM, Lloyd RM, Marcus CL and Vaughn BV for the American Academy of Sleep Medicine. The AASM Manual for the Scoring of Sleep and Associated Events: Rules, Terminology and Technical Specifications, Version 2.2. www.aasmnet.org. Darien, Illinois: American Academy of Sleep Medicine; 2015.
3. Iber C, Ancoli-Israel S, Chesson A, Quan S. The AASM manual for the scoring of sleep and associated events. Westchester, IL: American Academy of Sleep Medicine; 2007.
4. Sleep-related breathing disorders in adults: recommendations for syndrome definition and measurement techniques in clinical research. The Report of an American Academy of Sleep Medicine Task Force. Sleep. 1999; 22 (5): 667-689.
5. Berry RB, Budhiraja R, Gottlieb DJ, et al. Rules for scoring respiratory events in sleep: update of the 2007 AASM Manual for the Scoring of Sleep and Associated Events. Deliberations of the Sleep Apnea Definitions Task Force of the American Academy of Sleep Medicine. J Clin Sleep Med. 2012; 8 (5): 597-619.
6. Anderer P, Ross M, Cerny A, Shaw E. Automated Scoring of Sleep and Associated Events. Adv Exp Med Biol. 2022; 1384: 107-130.
7. Ruehland WR, Rochford PD, O'Donoghue FJ, Pierce RJ, Singh P, Thornton AT. The new AASM criteria for scoring hypopneas: impact on the apnea hypopnea index. Sleep. 2009; 32 (2): 150-157.
8. El Shayeb M, Topfer LA, Stafinski T, Pawluk L, Menon D. Diagnostic accuracy of level 3 portable sleep tests versus level 1 polysomnography for sleep-disordered breathing: a systematic review and meta-analysis. CMAJ. 2014; 186 (1): E25-51.
9. Bianchi MT, Goparaju B. Potential Underestimation of Sleep Apnea Severity by At-Home Kits: Rescoring In-Laboratory Polysomnography Without Sleep Staging. J Clin Sleep Med. 2017; 13 (4): 551-555.
10. Van Pee B, Massie F, Vits S, et al. A multicentric validation study of a novel home sleep apnea test based on peripheral arterial tonometry. Sleep. 2022; 45 (5).
11. Massie F, Mendes de Almeida D, Dreesen P, Thijs I, Vranken J, Klerkx S. An Evaluation of the NightOwl Home Sleep Apnea Testing System. J Clin Sleep Med. 2018; 14 (10): 1791-1796.
12. Bakker JP, Ross M, Vasko R, et al. Estimating sleep stages using cardiorespiratory signals: validation of a novel algorithm across a wide range of sleep-disordered breathing severity. J Clin Sleep Med. 2021.
13. Radha M, Fonseca P, Moreau A, et al. Sleep stage classification from heart-rate variability using long short-term memory neural networks. Sci Rep. 2019; 9 (1): 14149.
14. Fonseca P, van Gilst MM, Radha M, et al. Automatic sleep staging using heart rate variability, body movements, and recurrent neural networks in a sleep disordered population. Sleep. 2020.
15. Sun H, Ganglberger W, Panneerselvam E, et al. Sleep staging from electrocardiography and respiration with deep learning. Sleep. 2020; 43 (7).
16. Hedner J, White DP, Malhotra A, et al. Sleep staging based on autonomic signals: a multi-center validation study. J Clin Sleep Med. 2011; 7 (3): 301-306.
17. Beattie Z, Oyang Y, Statan A, et al. Estimation of sleep stages in a healthy adult population from optical plethysmography and accelerometer signals. Physiol Meas. 2017; 38 (11): 1968-1979.
18. Li Q, Li Q, Liu C, Shashikumar SP, Nemati S, Clifford GD. Deep learning in the cross-time frequency domain for sleep staging from a single-lead electrocardiogram. Physiol Meas. 2018; 39 (12): 124005.
19. Massie F, Van Pee B, Vits S, Verbraecken J, Bergmann J. Phenotyping REM OSA by means of peripheral arterial tone-based home sleep apnea testing and polysomnography: A critical assessment of the sensitivity and specificity of both methods. J Sleep Res. 2022; 31 (2): e13481.
20. Pillar G, Bar A, Shlitner A, Schnall R, Shefy J, Lavie P. Autonomic arousal index: an automated detection based on peripheral arterial tonometry. Sleep. 2002; 25 (5): 543-549.
21. Li A, Chen S, Quan SF, Powers LS, Roveda JM. A deep learning-based algorithm for detection of cortical arousal during sleep. Sleep. 2020; 43 (12).
22. Olsen M, Schneider LD, Cheung J, et al. Automatic, electrocardiographic-based detection of autonomic arousals and their association with cortical arousals, leg movements, and respiratory events in sleep. Sleep. 2018; 41 (3).
23. Punjabi NM, Shifa N, Dorffner G, Patil S, Pien G, Aurora RN. Computer-Assisted Automated Scoring of Polysomnograms Using the Somnolyzer System. Sleep. 2015; 38 (10): 1555-1566.
24. Chen L-C, Papandreou G, Schroff F, Adam H. Rethinking Atrous Convolution for Semantic Image Segmentation. 2017.
25. Fonseca P, Aarts RM, Foussier J, Long X. A novel low-complexity post-processing algorithm for precise QRS localization. Springerplus. 2014; 3: 376.
26. Berry RB, Quan SF, Abreu AR, et al. The AASM manual for the scoring of sleep and associated events: rules, terminology and technical specifications, version 2.6. Darien, Illinois: American Academy of Sleep Medicine; 2020.
27. Bakker JP, Ross M, Cerny A, et al. Scoring sleep with artificial intelligence enables quantification of sleep stage ambiguity: Hypnodensity based on multiple expert scorers and auto-scoring. Sleep. 2022.
28. Mathur R, Douglas NJ. Frequency of EEG arousals from nocturnal sleep in normal subjects. Sleep. 1995; 18 (5): 330-333.
29. Koo TK, Li MY. A Guideline of Selecting and Reporting Intraclass Correlation Coefficients for Reliability Research. J Chiropr Med. 2016; 15 (2): 155-163.
30. Bland JM, Altman DG. Measuring agreement in method comparison studies. Stat Methods Med Res. 1999; 8 (2): 135-160.
31. Kishore K, Mahajan R. Understanding Superiority, Noninferiority, and Equivalence for Clinical Trials. Indian Dermatol Online J. 2020; 11 (6): 890-894.
32. Newcombe RG. Two-sided confidence intervals for the single proportion: comparison of seven methods. Stat Med. 1998; 17 (8): 857-872.
33. Simel DL, Samsa GP, Matchar DB. Likelihood ratios with confidence: sample size estimation for diagnostic test studies. J Clin Epidemiol. 1991; 44 (8): 763-770.
34. Cohen J. A Coefficient of Agreement for Nominal Scales. Educational and Psychological Measurement. 1960; 20 (1): 37-46.
35. Benjamini Y, Yekutieli D. The Control of the False Discovery Rate in Multiple Testing Under Dependency. Ann Stat. 2001; 29.
36. Epstein LJ, Kristo D, Strollo PJ, Jr., et al. Clinical guideline for the evaluation, management and long-term care of obstructive sleep apnea in adults. J Clin Sleep Med. 2009; 5 (3): 263-276.
37. Malhotra A, Younes M, Kuna ST, et al. Performance of an automated polysomnography scoring system versus computer-assisted manual scoring. Sleep. 2013; 36 (4): 573-582.
38. Kuna ST, Benca R, Kushida CA, et al. Agreement in computer-assisted manual scoring of polysomnograms across sleep centers. Sleep. 2013; 36 (4): 583-589.
39. Magalang UJ, Chen NH, Cistulli PA, et al. Agreement in the scoring of respiratory events and sleep among international sleep centers. Sleep. 2013; 36 (4): 591-596.
40. Malhotra A, Owens RL. What is central sleep apnea? Respir Care. 2010; 55 (9): 1168-1178.
41. Roberts EG, Raphelson JR, Orr JE, LaBuzetta JN, Malhotra A. The Pathogenesis of Central and Complex Sleep Apnea. Curr Neurol Neurosci Rep. 2022; 22 (7): 405-412.
42. Perslev M, Darkner S, Kempfner L, Nikolic M, Jennum PJ, Igel C. U-Sleep: resilient high-frequency sleep staging. NPJ Digit Med. 2021; 4 (1): 72.
43. Bonnet MH, Doghramji K, Roehrs T, et al. The scoring of arousal in sleep: reliability, validity, and alternatives. J Clin Sleep Med. 2007; 3 (2): 133-145.
44. Basner M, Griefahn B, Muller U, Plath G, Samel A. An ECG-based algorithm for the automatic identification of autonomic activations associated with cortical arousal. Sleep. 2007; 30 (10): 1349-1361.
45. Sforza E, Jouny C, Ibanez V. Cardiac activation during arousal in humans: further evidence for hierarchy in the arousal response. Clin Neurophysiol. 2000; 111 (9): 1611-1619.
46. Sforza E, Juony C, Ibanez V. Time-dependent variation in cerebral and autonomic activity during periodic leg movements in sleep: implications for arousal mechanisms. Clin Neurophysiol. 2002; 113 (6): 883-891.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. The computer program product may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A method for detecting sleep disordered breathing (SDB-) events, comprising:
receiving an arousal signal representative of an occurrence of an autonomic arousal;
receiving an SDB-signal representative of an occurrence of an SDB-event; and
generating a confirmation signal representing confirming that the SDB-signal is representative of the occurrence of the SDB-event based on the arousal signal.

2. The method according to claim 1, comprising generating a further confirmation signal representing confirming that the autonomic arousal is caused by the SDB-event based on the arousal signal and the SDB-signal.

3. The method according to any one of the preceding claims, wherein the arousal signal is based on at least one of a cardiac signal generated by a cardiac sensor and a respiratory signal generated by a respiratory sensor,
wherein the cardiac signal represents a cardiac parameter,
wherein the respiratory signal represents a respiratory parameter other than respiratory flow and SpO2,
wherein the SDB-signal is based on the respiratory signal.

4. The method according to any one of the preceding claims, comprising
receiving a sleep stage signal representative of a sleep stage of the subject;
calculating a total sleep time of the user based on the sleep stage signal; and
determining an apnea-hypopnea index (AHI) based on the SDB-signal, the confirmation signal and the total sleep time.

5. A machine-learning model for generating an arousal signal for use in the method according to any one of claims 1-4, the machine-learning model comprising:
at least one of a cardiac feature extraction module and a respiratory feature extraction module,
wherein the cardiac feature extraction module is configured to generate an estimated cardiac parameter based on a cardiac signal representative of a cardiac parameter of a subject,
wherein the respiratory feature extraction module is configured to generate an estimated respiratory parameter based on a respiratory signal representative of a respiratory parameter of the subject;
an arousal detection module configured to generate an estimated arousal probability based on at least one of the estimated cardiac parameter and the respiratory parameter,
wherein the machine-learning model is configured to generate the arousal signal based on the estimated arousal probability.

6. The machine-learning model of claim 5, wherein at least one of the cardiac feature extraction module, the respiratory feature extraction module and the arousal detection module comprises at least one residual convolutional network block,
wherein the at least one residual convolutional network block comprises:
a stack of at least two one-dimensional convolutions, the at least two one-dimensional convolutions having an exponentially increasing dilation rate, and
at least one skip connection.

7. The machine-learning model of claim 6, comprising a dense layer configured to receive an output from the stack of at least two one-dimensional convolutions.

8. The machine-learning model of any of claims 5-7, comprising both the cardiac feature extraction module and the respiratory feature extraction module,
wherein each of the cardiac feature extraction module, the respiratory feature extraction module and the arousal detection module comprises at least one residual convolutional network block,
wherein each of the residual convolutional network blocks comprises:
a stack of at least two one-dimensional convolutions, the at least two one-dimensional convolutions having an exponentially increasing dilation rate, and
at least one skip connection.

9. The machine-learning model of any one of claims 5-8, wherein the machine learning model has been trained by at least one of:
training the cardiac feature extraction module by deriving an instant heart rate signal from reference ECG data obtained in parallel to the cardiac signal;
training the cardiac feature extraction module, the respiratory feature model and the arousal detection module end-to-end using cortical arousal, derived from reference EEG data obtained in parallel to the cardiac signal and the respiratory signal, as a target.

10. Detecting device for detecting SDB-events, comprising
a sensor system; and
a processing unit connected to the sensor system,
wherein the sensor system is configured to generate an arousal signal and is configured to generate an SDB-signal,
wherein the arousal signal is representative of an occurrence of an autonomic arousal,
wherein the SDB-signal is representative of an occurrence of an SDB-event;
wherein the processing unit is configured to perform the method according to any one of claims 1-4.

11. Detecting device according to claim 10, wherein the sensor system comprises a first sensor and a second sensor,
wherein the first sensor is configured to measure a cardiac parameter of the subject, wherein the second sensor is configured to measure a respiratory parameter of the subject other than respiratory flow and SpO2,
wherein the sensor system is configured to generate the arousal signal based on the cardiac parameter and the respiratory parameter.

12. Detecting device according to claim 11, comprising the machine-learning model of any one of claims 5-9.

13. A home sleep apnea test device comprising the detecting device according to any one of claims 11-12.

14. A computer program product, comprising instructions which, when executed by a processing unit, cause the processing unit to carry out at least one of the method of claims 1-4 and the machine-learning model of any one of claims 5-9.

15. A computer-readable storage medium comprising at least one of instructions which, when executed by a processing unit, cause the processing unit to carry out the method of any one of claims 1-4, and the machine-learning model of any one of claims 5-9.
